Europäisches Patentamt

European Patent Office

Office européen des brevets

(11) Veröffentlichungsnummer: **0 666 438 A1**

## (12) EUROPÄISCHE PATENTANMELDUNG

(21) Anmeldenummer: **94120725.0**

(22) Anmeldetag: **27.12.94**

(51) Int. Cl.⁶: **F16J 15/53**, F16K 1/34, F16K 13/10

(30) Priorität: **04.02.94 SE 9400366**

(43) Veröffentlichungstag der Anmeldung:
**09.08.95 Patentblatt 95/32**

(84) Benannte Vertragsstaaten:
**CH DE ES FR GB IT LI NL SE**

(71) Anmelder: **Siemens Elema AB**
**Röntgenvägen 2**
**S-171 95 Solna 1 (SE)**

(72) Erfinder: **Lekholm, Anders**
**Gnejsvägen 4**
**S-161 93 Bromma (SE)**

(54) **Ventil zum Steuern eines Gas- oder Flüssigkeitsflusses und die Verwendung des Ventils bei einer implantablen Medikamentpumpe.**

(57) Die Erfindung bezieht sich auf ein Ventil zum Steuern eines Gas-oder Flüssigkeitsflusses und die verwendung des Ventils bei einer implantablen Medikamentpumpe. Das Ventil ist mit einem Ein- und einem Auslass mit einem Ventilsitz, einem gegenüber dem Ventilsitz verschiebbaren Ventilteil und einer Dichtung dazwischen versehen, wobei das Ventilteil in einer Lage die Dichtung gegen den Ventilsitz drückt und somit den fluß unterbricht. Um ein Ventil dieser Art zu erhalten, das im Aufbau einfach ist und während einer verhältnismässig langen Zeitperiode ein extrem hohes Dichtungsvermögen aufweist, wird erfindungsgemäß vorgeschlagen, daß als Dichtung (12,14,15,17,20,27) zumindest teilweise eine Flüssigkeit mit derartigen Eigenschaften dient, daß sie in der Flüssigkeit bzw. dem Gas, die bzw. das durch Ventil (13,17,24) fließt, unlöslich ist.

FIG 3

EP 0 666 438 A1

Rank Xerox (UK) Business Services
(3.10/3.09/3.3.4)

Die Erfindung bezieht sich auf ein Ventil zum Steuern eines Gas- oder Flüssigkeitsflusses mit einem Ein- und einem Auslaß mit einem Ventilsitz, einem gegenüber dem Ventilsitz verschiebbaren Ventilteil und einer Dichtung dazwischen, wobei das Ventilteil in einer Lage die Dichtung gegen den Ventilsitz drückt und somit den Fluß unterbricht.

Es ist bei konventionellen Ventilen ein wohlbekanntes Problem, dass sowohl der Ventilsitz als auch das Ventilteil im Bereich der dichtenden Oberflächen Imperfektionen aufweisen oder Schäden erhalten können, die zu einer Leckage führen können. Nach einiger Zeit kann auch eine elastische Dichtung oder Packung erhärten oder deformiert werden, so dass ein anfangs wohl funktionierendes Ventil mit der Zeit unbrauchbar wird. In Verbindung mit Vorrichtungen, bei denen eine hohe Präzision bei einer Dosierung von Gasen oder Flüssigkeiten erforderlich ist, insbesondere dann, wenn das Ventil auch sehr geringe Mengen durchlassen soll, ist es von größter Bedeutung, dass dieses Ventil auch nach einer längeren Verwendungszeit gut abdichtet.

Ein Gerät, dessen Ventile ein sehr hohes Dichtungsvermögen aufweisen müssen, ist eine implantierbare Medikamentendosierpumpe für Insulindosierungen, bei der das Medikament in sehr kleinen Dosen mit einem Volumen von etwa 1 µl abgegeben werden soll. Ein solches Gerät, das mit einem konventionellen Ventil der eingangs genannten Art versehen ist, ist durch die US-PS 4 883 467 bekannt.

Der Erfindung liegt die Aufgabe zugrunde, ein Ventil der eingangs genannten Art zu schaffen, das im Aufbau einfach ist und während einer verhältnismässig langen Zeitperiode ein extrem hohes Dichtungsvermögen aufweist.

Diese Aufgabe ist erfindungsgemäss dadurch gelöst, dass als Dichtung zumindest teilweise eine Flüssigkeit mit derartigen Eigenschaften dient, dass sie in der Flüssigkeit bzw. dem Gas, die bzw. das durch das Ventil fließt, unlöslich ist. Wenn ein Ventil mit einer derartigen Dichtung schliesst, erfolgt eine Deformierung der Flüssigkeit, wobei eine extrem gute Dichtung erhalten wird. Die Deformierungskraft der Dichtung wird etwa gleich gross oder kleiner als bei einer Dichtung, die aus einem elastischen Material hergestellt ist. Wenn das Ventil geöffnet wird, nimmt die Dichtung ihre ursprüngliche Form wieder an und ist somit frei von derartigen Alterungserscheinungen, denen üblicherweise eine konventionelle Dichtung ausgesetzt wird, da diese, wie beschrieben, mit der Zeit eine permanente Deformation erhält, was die abdichtende Funktion des Ventils gefährden kann.

In einer vorteilhaften Weiterbildung der Erfindung wird vorgeschlagen, daß die Dichtungsflüssigkeit einen Tropfen bildet, der zum Teil von einem im Ventil vorgesehenen Hohlraum umschlossen ist. Eine solche Dichtung eignet sich für ein Ventil, das den Auslass einer Gas- oder Flüssigkeitskammer, bei z.B. einem Medikamentendosiergerät, dessen der Auslaß einen sehr kleinen Durchmesser aufweist, dichten soll.

In einer vorteilhaften Ausbildung der Erfindung wird vorgeschlagen, daß der Ventilsitz oder das Ventilteil mit einem ringförmigen Hohlraum versehen ist, der die Dichtungsflüssigkeit zum Teil umschliesst. Eine derartige Flüssigkeitsdichtung eignet sich für ein Ventil mit einem Auslaß für Gas oder Flüssigkeit, der einen im Vergleich zu dem oben erwähnten Auslass grösseren Durchmesser aufweist.

Nach der Erfindung soll die Dichtungsflüssigkeit eine solche Oberflächenspannung und Form aufweisen, dass der hierdurch erzeugte interne Druck grösser ist als der Druck desjenigen Gases bzw. derjenigen Flüssigkeit, das bzw. die das Ventil abdichten soll. In dieser tropfenförmigen Flüssigkeitsdichtung entsteht aufgrund der Oberflächenspannung $T$ der Flüssigkeit ein Überdruck $P$. Dabei besteht folgender Zusammenhang: $P = 2\ T/r$, bei der $r$ der Radius des Tropfens ist.

Mit realistischen Werten in dieser Formel und mit einem Durchmesser des Auslaßes der Gas- oder Flüssigkeitskammer von einigen zehnteln Millimetern kann die Oberflächenspannung den Tropfen bis auf einen für viele Anwendungen ausreichenden Druck zusammenhalten.

Da die Oberflächenspannung u.a. in der Grenzfläche zwischen zwei Flüssigkeiten entsteht, muss die Dichtungsflüssigkeit mit Rücksicht auf die Flüssigkeit, die herausgepumpt werden soll, gewählt werden. Bei einer Insulinpumpe kann die Dichtungsflüssigkeit vorzugsweise eine polymere Flüssigkeit sein.

Die Dichtungsflüssigkeit kann in Verbindung mit weiteren Verwendungen vorzugsweise aus Quecksilber bestehen.

Bei einem Ventil der eingangs genannten Art, bei dem das Ventilteil ein magnetisierbares Material aufweist, das von mindestens einem vorzugsweise am Ventilsitz angeordneten Magneten beeinflusst wird, wird nach der Erfindung vorgeschlagen, dass die Dichtungsflüssigkeit aus einer magnetischen Flüssigkeit besteht. Durch Auflegen einer magnetischen Flüssigkeit auf dem Ventilsitz und/oder auf dem Ventilteil wird eine perfekte Dichtung mit den vorhergehend erwähnten Vorteilen erhalten, auch wenn der Ventilsitz und/oder das Ventilteil kleine Schäden aufweisen.

Nach einer weiteren vorteilhaften Ausbildung der Erfindung wird vorgeschlagen, dass der Teil des Ventils, der keine magnetische Flüssigkeit aufweist, mit einer konventionellen Dichtung versehen ist. Auch bei einer solchen Ausführungsform wird eine sehr gute Dichtung mit einer langen Lebensdauer erhalten.

Die magnetische Flüssigkeit besteht nach der Erfindung aus einer Suspension von äusserst kleinen Partikeln aus einem magnetischen Material in einer Flüssigkeit. Das magnetische Material der Flüssigkeit ist derart pulverisiert und behandelt, dass dessen chemischen Eigenschaften normalerweise in der Flüssigkeit, in der das Material suspendiert ist, verborgen ist.

Weitere Vorteile und Einzelheiten ergeben sich aus den Unteransprüchen.

Die Erfindung ist nachfolgend anhand mehrerer in den Zeichnungen dargestellten Ausführungsbeispielen näher erläutert. Es zeigen:

FIG 1 einen Längsschnitt einer vergrößerten Medikamentenpumpe mit einem Ventil nach der Erfindung und

FIG 2 bis 6 eine weitere Vergrößerung des Auslaßsystems der Medikamentenpumpe nach der FIG 1 mit Ventilen nach der Erfindung in verschiedenen Ausführungen und Lagen.

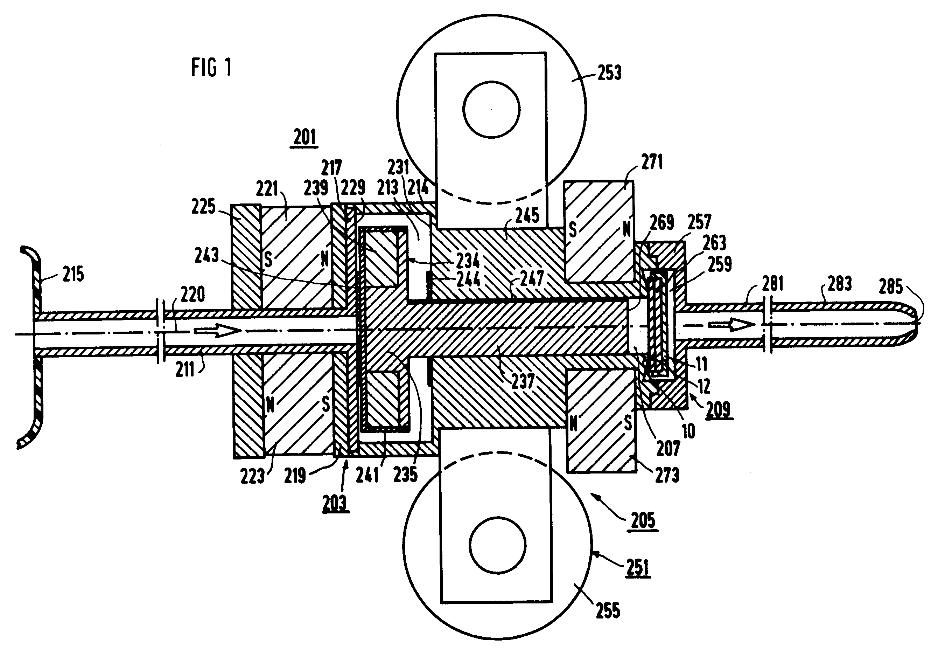

In FIG. 1 ist eine Vergrößerung einer implantierbaren Medikamentenpumpe 201 für ein Medikamentenfördervolumen von etwa 1 $\mu$l dargestellt.

Diese Medikamentenpumpe 201, die von der Type Kolbenpumpe ist, setzt sich im wesentlichen aus einem Einlaßsystem 203 mit magnetischer Rückstellfeder, einem Pumpsystem 205 mit Pumpkammer 207 und einem Auslaßsystem 209 mit integriertem Rückschlagventil zusammen.

Das Einlaßsystem 203 umfaßt einen Fluidtrakt, der eine Einlaßleitung 211 und eine zylindrische Einlaßkammer 213 in einem Gehäuse 214 besitzt. An die Einlaßleitung 211 ist ein Medikamentenreservoir 215 anschließbar, das vorzugsweise mit Unterdruck gegenüber der Umgebung, z.B. mit 300 mbar, beaufschlagt ist. Bei dem Reservoir 215 kann es sich insbesondere um einen Behälter mit Insulin handeln. Die Einlaßleitung 211 mündet über einen Flansch 217 in die Einlaßkammer 213.

Auf dem Endstück der Einlaßleitung 211 sitzt ein nichtmagnetisierbarer zylindrischer Tragkörper 219, an dem diametral zur Achse 220 zwei Permanentmagnete 221, 223 angebracht sind. Diese Magnete 221, 223 sind z.B. jeweils von zylindrischer oder quaderförmiger Gestalt und besitzen die eingezeichneten Polaritäten S-N und N-S.

In der Einlaßkammer 213 befindet sich ein axial verschiebbarer zylindrischer Anker 234. Dieser besitzt einen Ankerträger 235, der am rechten Ende in einen längeren Kolben 237 geringeren Durchmessers übergeht.

Im gezeigten Ausführungsbeispiel ist auf dem Ankerträger 235 ein ringförmiges Ankerteil 239 befestigt. Das Ankerteil 239 ist dabei über den Kern des in den Kolben 237 übergehenden Ankerträgers 235 gestülpt. Das Ankerteil 239 ist aus einem magnetisierbaren Material, beispielsweise Weicheisen, gefertigt. Der Ankerträger 235 ist teilweise und das Ankerteil 239 ist vollständig von einer zylindrischen Dose oder Kapsel 241 umgeben oder eingefaßt.

Die außerhalb der Einlaßkammer 213 plazierten Permanentmagnete 221, 223 üben auf den Anker 234 dauernd eine Kraft aus, die den Anker 234 samt Kolben 237 in Richtung auf die Einlaßleitung 211 hinzieht, die Einlaßöffnung im Flansch 217 dabei aber nicht verschließt. Für die Begrenzung dieser Rückziehbewegung kann ein Anschlag 243, z.B. in Form einer dünnen ringförmigen und zentral befestigten Platte, an der Stirnseite 229 des Flansches 217 vorgesehen sein. Ein weiterer Anschlag 244, z.B. ebenfalls in Form eines dünnen, axial plazierten Ringes, kann auf der anderen Stirnseite 231 der Einlaßkammer 213 angebracht sein. Befindet sich der Anker 234 an der linken Stirnseite 229, so ist der Kolben 237 in seiner Senkstellung oder Ruhelage.

Die beiden Permanentmagnete 221, 223 samt Tragkörper 219 und Rückschlußteil 225 sind vorzugsweise in Richtung der Längsachse 220 definiert verschiebbar, wodurch die Rückstellkraft auf den Anker 234 und damit den Kolben 237 kontinuierlich auf einen gewünschten Wert einstellbar ist.

Der Kolben 237 ist longitudinal in einem Zylinder 245 verschiebbar. Zwischen Zylinder 245 und Kolben 237 ist dabei ein Spalt 247, insbesondere ein Ringspalt, vorgesehen, durch den bei Rückstellung des Kolbens 237 in seine Ruhelage das flüssige Medikament von links nach rechts gefördert wird, wie später näher dargelegt ist.

Der Anker 234 mit ferromagnetischem Ankerteil 239 dient gleichzeitig auch als Anker für ein elektromagnetisches Antriebssystem 251, das am Zylinder 245 angeordnet ist. Dieses System weist zwei elektromagnetische Spulen 253, 255 auf. Bei deren Erregung mittels eines Stromes wird ein magnetisches Feld erzeugt, das eine ausreichend große Kraftwirkung auf den Anker 234 bereitstellt. Dadurch wird der Anker 234 samt Kolben 237 in Richtung auf das Auslaßsystem 209 verschoben, und zwar bis der Anker 234 an der rechten Stirnseite 231 oder an dem Anschlag 244 anstößt.

Stromabwärts direkt hinter der Pumpkammer 207 befindet sich ein Ventil 13, umfassend einen Ventilsitz 10, ein gegenüber dem Ventilsitz 10 verschiebbares Ventilteil 11 und dazwischen eine Dichtung 12. Das Ventil 13 ist in einem Ansatzflansch 257, der eine Auslaßkammer 259 bildet, untergebracht.

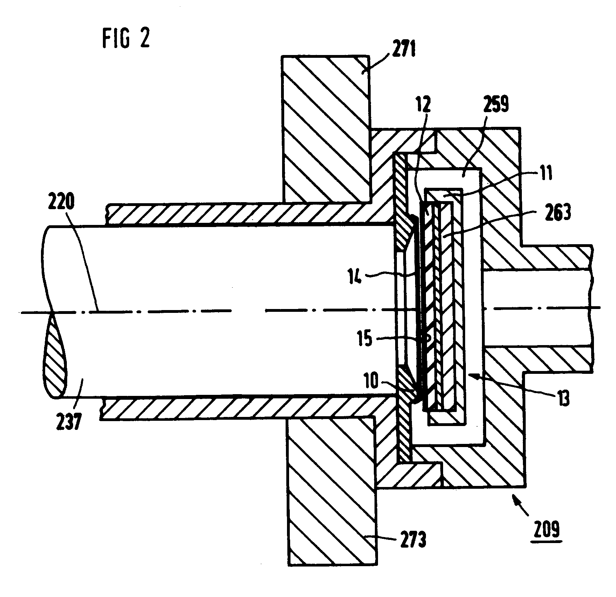

Das Ventilteil 11 ist mit einem Element 263 aus einem magnetisierbaren Material gefertigt und dient als Anker. Das Element 263 bildet zusammen mit zwei Dauermagneten 271 und 273, die im Zylinder 245 um die Zentrumachse 220 mit den angegebenen Polaritäten S-N bzw. N-S angebracht sind, ein magnetisches Federsystem. Das magnetische Federsystem 271, 273, 263 hält das Ventilteil 11 in einer Ruhestellung, bei der dieses Ventilteil 11 die Dichtung 12 gegen den Ventilsitz 10 drückt. Das Ventil 13 mit der Dichtung 12 gemäss der Erfindung wird später in Verbindung mit den FIG 2 bis 6 näher beschrieben.

An die Auslaßkammer 259 mit Ventilteil 11 schließt über den Ansatzflansch 257 eine Auslaßleitung 281 an, die in einen Katheter 283 mit Auslaßöffnung 285 übergeht. Bei der Implantation ist die Auslaßöffnung 285 an vorgegebener Stelle im Patienten positioniert.

Im Betrieb der Kolbenpumpe 201 wird der Kolben 237 durch die elektromagnetische Feldwirkung der Spulen 253, 255 aus der dargestellten Ruhelage nach rechts bewegt, wodurch im Medikament in der Pumpkammer 207 ein starker Überdruck entsteht. Sobald die durch den Überdruck auf das bewegliche Ventilteil 11 ausgeübte Kraft die Rückstellkraft der magnetischen Feder 263, 271, 273 überschreitet, hebt sich das Ventilteil 11 mit seiner Dichtung 12 von dem Ventilsitz 10 ab. Zwischen dem Ventilteil 11 und dem Ventilsitz 12 bildet sich ein Ventilspalt. Das flüßige Medikament, z.B. Insulin, wird über die Auslaßleitung 281 in den Katheter 283 gedrückt. Der Katheter 283 ist dabei direkt an der Auslaßkammer 259 angeschlossen. Das in der Pumpkammer 207 enthaltene flüssige Medikament durchströmt dabei den Ventilspalt, die Auslaßkammer 259 und den Katheter 283 in Pfeilrichtung.

Wird der Kolben 237 nach Abschalten der Spulen 253, 255 wieder in seine in FIG 1 gezeigte Senkstellung zurückbewegt, so wird in der Pumpkammer 207 ein Unterdruck erzeugt. Ist dessen absoluter Wert kleiner als derjenige im Medikamentenspeicher 215, so entsteht ein Druckgefälle. Das flüssige Medikament wird dabei gegen den Unterdruck im Medikamentenspeicher 215 durch den stärkeren Unterdruck in der Pumpkammer 207 in diese hineingesaugt. Dabei durchströmt es den Spalt 247 zwischen dem Kolben 237 und dem Zylinder 245. Das Ventilteil 11 und der Ventilsitz 10 wirken dabei als Rückschlagventil. Nach dem besagten Abschalten der Spulen 253, 255 wird der Anker 234 samt Kolben 237 durch die Permanentmagnete 221, 223, die auf das Ankerteil 239 wirken, in seine Senkstellung oder Ruhelage zurückgeführt. Wenn dem Patienten wieder Insulin zugeführt werden soll, wird das beschriebene Pumpverfahren wiederholt.

In der FIG 2, die eine weitere Vergrößerung des in der FIG 1 gezeigten Auslaßsystems 209 der Medikamentenpumpe 201 ist, ist das Ventil 13 deutlich dargestellt. In der FIG ist das Ventil 13, im Gegensatz zum Ventil 13 in der FIG 1, in eine in Verbindung mit dieser FIG 1 beschriebenen, offenen Lage gezeigt. In dieser offenen Lage befindet sich das Ventilteil 11 in einer Position, bei der dessen Dichtung 12 nicht gegen den Ventilsitz dicht anliegt, sondern den Abstand einer Spaltenöffnung zum Ventilsitz aufweist, so daß das fließende Medikament durch die Ventilspalte in die Auslaßkammer 259 strömen kann. Das Medikament wird danach über die Auslaßleitung 281 in den in Verbindung mit der FIG 1 gezeigten und beschriebenen Katheter 283 hineinbefördert.

Der Ventilsitz 10 des Ventils 13 ist mit einer Schicht aus einer magnetischen Flüssigkeit, die in der FIG 2 mit einem Strich 14 dargestellt ist, versehen. Die magnetische Flüssigkeit haftet am Ventilsitz 10, da zwei Dauermagnete 271 und 273 wie beschrieben, an dem Ventilsitz 10 angeordnet sind. Da das Ventilteil 11 ein Element 263 aus einem magnetisierbaren Material beinhaltet, ist es auch möglich, die Dichtung 12 des Ventilteils 11 mit einer magnetischen Flüssigkeit, die mit einem Strich 15 dargestellt ist, zu beschichten.

In dem Fall, wo nur eine der Schichten 14 oder 15 auf eines der erwähnten Teile 10 bzw. 11 aufgelegt wird, ist der Teil des Ventils 13, der nicht mit einer magnetischen Flüssigkeit beschichtet ist, mit einer konventionellen Dichtung versehen. Die magnetische Flüssigkeit 14, 15 besteht aus einer Suspension von äußerst kleinen Partikeln aus einem magnetischen Material in einer viskosen organischen Flüssigkeit. Die magnetische Flüssigkeit 14, 15 weist derartige Eigenschaften auf, daß sie in der Flüssigkeit, die das Ventil durchströmt, unlöslich ist. Die magnetische Flüssigkeit ist in Bezug auf die Flüssigkeit, mit der sie in Verbindung kommt, auch inert. Durch Beschichten des Ventilsitzes 10 und/oder der Dichtung 12 des Ventilteiles 11 mit einer magnetischen Flüssikgeit wird beim Schließen des Ventils 13 eine extrem gute Dichtung auch dann erhalten, wenn der Ventilsitz 10 und/oder die Dichtung Unebenheiten oder, wenn die Dichtung 12, die in der Regel ein Membran ist, Alterungserscheinungen aufweisen. Wenn das Ventilteil 11 gegen den Ventilsitz 10 drückt, wird die Schicht bzw. werden die Schichten 14, 15 der magnetischen Flüssigkeit deformiert. Auf diese Weise wird eine sehr gute Dichtung erhalten.

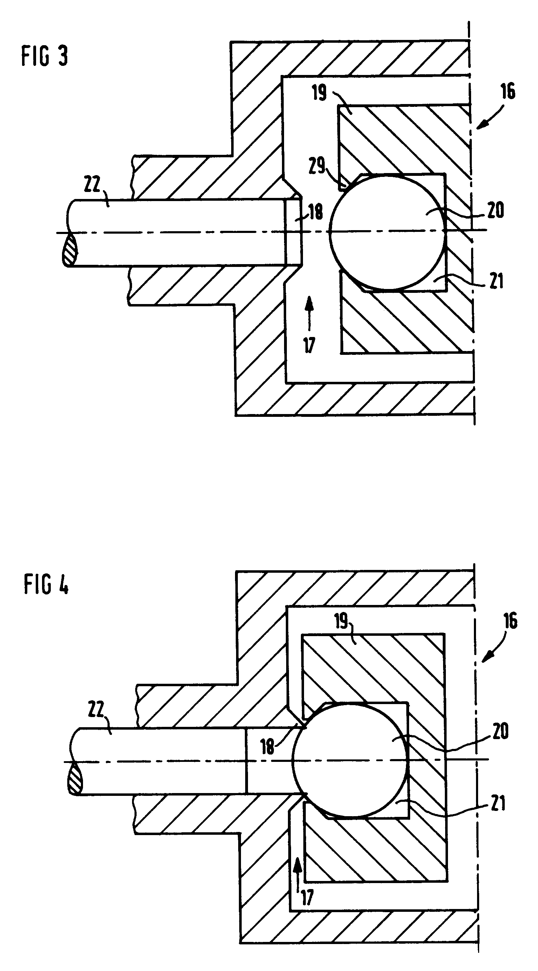

In der FIG 3 ist eine Vergrößerung eines Auslaßsystems 16 mit einem Ventil 17 dargestellt, bei dem das Auslaßsystem ein Teil einer Medikamentenpumpe nach FIG 1 sein kann. Das Auslaßsystem kann aber auch ein Teil eines Gerätes zum Dosieren von Gasen oder Flüssigkeiten sein, bei dem die Pumpe nicht notwendigerweise mittels eines magnetischen Federsystems angetrieben wird. Auch dieses Ventil 17 umfaßt einen Ventilsitz 18, ein gegenüber dem Ventilsitz 18 verschiebbares Ventilteil 19 und eine Dichtung 20. Die Dichtung 20 besteht aus einer Flüssigkeit, die einen Tropfen bildet, der zum Teil von einem im Ventilteil 19 vorgesehenen Hohlraum 21 umschlossen ist. Da der Hohlraum 21 etwas kleiner als die Größe des Tropfens ist und mit einem Falz 29 versehen ist, wird der Tropfen automatisch am Platz gehalten. Ähnlich wie die früher erwähnte magnetische Flüssigkeit, weist diese Dichtungsflüssigkeit derartige Eigenschaften auf, daß sie in der Flüssigkeit bzw. in das Gas, die bzw. das durch das Ventil strömt, unlöslich ist. Die tropfenförmige Dichtungsflüssigkeit 20 weist eine derartige Oberflächenspannung auf, daß der hierdurch erzeugte interne Druck größer als der Druck desjenigen Gases bzw. derjenigen Flüssigkeit, das bzw. die das Ventil abdichten soll, ist. In dieser FIG 3 ist das Ventil in einer offenen Lage gezeigt, bei der ein Pumpenkolben 22 ein Medium beeinflußt hat, dieses gegen die tropfenförmige Dichtungsflüssigkeit 20 zu drücken, derart, daß die Dichtungsflüssigkeit zusammen mit dem Ventilteil 19 sich vom Ventilsitz 18 abheben, so daß eine Spaltenöffnung für das Medium gebildet wird. Wenn der Kolben danach in die entgegengesetzte Richtung verschoben wird, bildet sich vor dem Kolben ein Unterdruck, wobei das Ventilteil 19 und vor allem die tropfenförmige Dichtungsflüssigkeit 20 gegen den Ventilsitz 18 gedrückt wird, so daß die Flüssigkeit 20, wie es in der FIG 4 gezeigt ist, danach geformt wird. Hierdurch wird eine extrem gute Dichtung erhalten. Wenn das Ventil 17 wieder geöffnet wird, nimmt die Dichtungsflüssigkeit 20 ihre ursprüngliche Tropfenform wieder an. Die Dichtungsflüssigkeit 20 kann vorzugsweise ein Polymer sein. Die Dichtungsflüssigkeit kann auch aus Quecksilber bestehen.

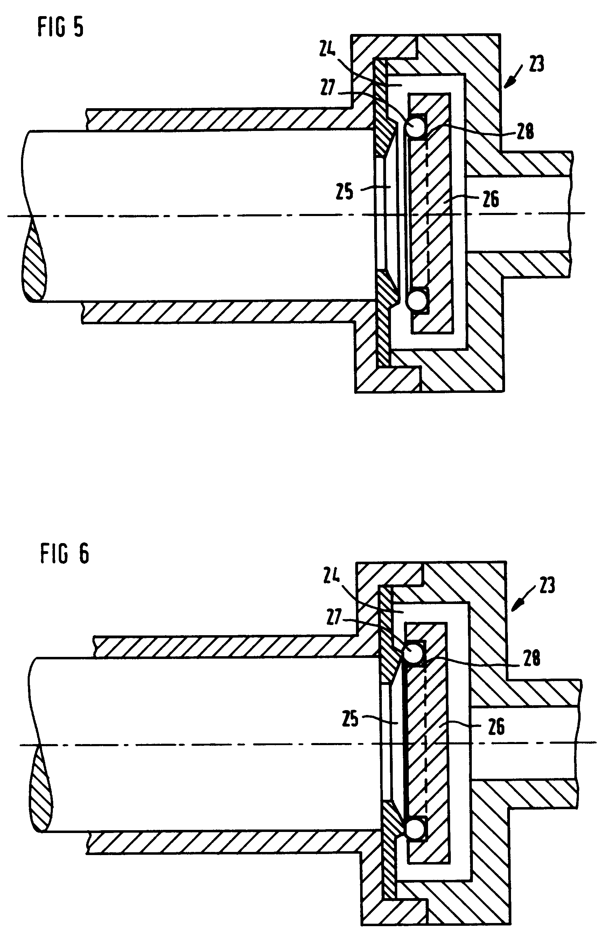

In der FIG 5 und 6 ist eine Vergrößerung eines weiteren Auslaßsystems 23 mit einem Ventil 24 gezeigt. Das Auslaßsystem 23 kann ein Teil eines Gerätes zum Dosieren von Gasen oder Flüssigkeiten sein. Das Ventil 24 umfaßt einen Ventilsitz 25, ein gegenüber dem Ventilsitz 25 verschiebbares Ventilteil 26 und eine Dichtung 27. Das Ventilteil 26 ist mit einem ringförmigen Hohlraum 28 versehen, der die Dichtung 27 zum Teil umschließt. Auch diese Dichtung 27, die im Profil vorzugsweise ovalförmig ist, besteht aus einer Flüssigkeit, z.B. Quecksilber oder Polymer. In der FIG 5 ist das Ventil 24 in einer offenen Lage und in der FIG 6 ist das Ventil 24 in einer geschlossenen Lage gezeigt. In der geschlossenen Lage wird die ovalförmige Flüssigkeit deformiert und dichtet dadurch gegen den Ventilsitz 25 extrem gut ab. Der ringförmige Hohlraum 28 mit der Dichtungsflüssigkeit 27 kann nach der Erfindung auch im Ventilsitz 25 angeordnet sein. In einem solchen Ausführungsbeispiel kann das Ventilteil 26 mit einer konventionellen Dichtung versehen sein. Eine solche Flüssigkeitsdichtung nach den FIG 5 und 6 eignet sich für ein Ventil, das einen Auslaß für eine Gas- oder Flüssikgeitskammer mit einem verhältnismäßig großen Durchmesser abdichten soll.

Es ist wesentlich, daß die Ventile, die den Fluß in einer Pumpe steuern, mit einer minimalen Verwendung von Energie sowohl beim Öffnen als auch beim Schließen der Ventile arbeiten. Dies ist von großer Bedeutung, insbesondere dann, wenn eine hohe Präzision bei der Dosierung erforderlich ist und/oder wenn kleine Fördervolumen gepumpt werden sollen. Durch die hier beschriebene Erfindung kann eine Dichtung mit geringst möglicher Kraft deformiert werden, so daß sie perfekt abdichtet und dadurch Leckage im Bereich des Arbeitsdruckgebietes verhindert.

Bezugszeichenliste

| | |
|---|---|
| 10, 18, 25 | Ventilsitz |
| 11, 19, 26 | Ventilteil |
| 12, 27 | Dichtung |
| 13, 17, 24 | Ventil |
| 14, 15 | Schicht, magnetische Flüssigkeit |
| 16, 23, 209 | Auslaßsystem |
| 20 | Dichtung,tropfenförmige Dichtungsflüssigkeit |
| 21, 28 | Hohlraum |
| 22 | Pumpenkolben |
| 29 | Falz |
| 201 | Medikamentenpumpe |
| 203 | Einlaßsystem |
| 205 | Pumpsystem |
| 207 | Pumpkammer |
| 211 | Einlaßleitung |
| 213 | Einlaßkammer |
| 214 | Gehäuse |
| 215 | Medikamentenreservoir |
| 217 | Flansch |
| 219 | Tragkörper |
| 220 | Achse, Längsachse |
| 221, 223 | Permanentmagnet |
| 225 | Rückschlußteil |
| 229, 231 | Stirnseite |
| 234 | Anker |
| 235 | Ankerträger |
| 237 | Kolben |
| 239 | Ankerteil |
| 241 | zylindrische Dose, Kapsel |
| 243, 244 | Anschlag |
| 245 | Zylinder |
| 247 | Spalt |
| 251 | Antriebssystem |
| 253, 255 | elektromagnetische Spule |
| 257 | Ansatzflansch |
| 259 | Auslaßkammer |
| 263 | Element, magnetisches Federsystem |

| 271, 273 | Dauermagnet, magnetisches Federsystem |
| 281 | Auslaßleitung |
| 283 | Katheter |
| 285 | Auslaßöffnung |

**Patentansprüche**

1. Ventil zum Steuern eines Gas- oder Flüssigkeitsflußes mit einem Ein- und einem Auslaß mit einem Ventilsitz, einem gegenüber dem Ventilsitz verschiebbaren Ventilteil und einer Dichtung dazwischen, wobei das Ventilteil in einer Lage die Dichtung gegen den Ventilsitz drückt und somit den Fluß unterbricht, **dadurch gekennzeichnet,** daß als Dichtung (12, 14, 15, 17, 20, 27) zumindest teilweise eine Flüssigkeit mit derartigen Eigenschaften dient, daß sie in der Flüssigkeit bzw. dem Gas die bzw. das durch das Ventil (13, 17, 24) fließt, unlöslich ist.

2. Ventil nach Anspruch 1, **dadurch gekennzeichnet,** daß die Dichtungsflüssigkeit (14, 15, 17, 20,27) eine solche Oberflächenspannung und Form aufweist, daß der hierdurch erzeugte interne Druck größer ist als der Druck desjenigen Gases bzw. derjenigen Flüssigkeit, das bzw. die das Ventil (13, 17, 24) abdichten soll.

3. Ventil nach einem der Ansprüche 1 oder 2, **dadurch gekennzeichnet,** daß die Dichtungsflüssigkeit (20) einen Tropfen bildet, der zum Teil von einem im Ventilteil (19) vorgesehenen Hohlraum (21) umschlossen ist.

4. Ventil nach Anspruch 1 oder 2, **dadurch gekennzeichnet,** daß der Ventilsitz (25) oder das Ventilteil (26) mit einem ringförmigen Hohlraum (28) versehen ist, der die Dichtungsflüssigkeit (27) zum Teil umschließt.

5. Ventil nach einem der Ansprüche 1 bis 4, **dadurch gekennzeichnet,** daß die Dichtungsflüssigkeit (20, 27) aus Quecksilber besteht.

6. Ventil nach einem der Ansprüche 1 bis 4, **dadurch gekennzeichnet,** daß die Dichtungsflüssigkeit (20, 27) eine polymere Flüssigkeit ist.

7. Ventil nach Anspruch 1, bei dem das Ventilteil ein magnetisierbares Material aufweist, das von mindestens einem vorzugsweise am Ventilsitz angeordneten Magneten beeinflußt wird, **dadurch gekennzeichnet,** daß die Dichtungsflüssigkeit (14, 15) aus einer magnetischen Flüssigkeit besteht.

8. Ventil nach Anspruch 7, **dadurch gekennzeichnet,** daß der Ventilsitz (10) und/oder das Ventilteil (11) mit einer magnetischen Flüssigkeit (14, 15) versehen wird.

9. Ventil nach einem der Ansprüche 7 oder 8, **dadurch gekennzeichnet,** daß der Teil (10, 11) des Ventils (13), der keine magnetische Flüssigkeit (14, 15) aufweist, mit einer konventionellen Dichtung versehen ist.

10. Ventil nach einem der Ansprüche 7 bis 9, **dadurch gekennzeichnet,** dass die magnetische Flüssigkeit (14, 15) aus einer Suspension von äußerst kleinen Partikeln aus einem magnetischen Material in einer Flüssigkeit besteht.

11. Ventil nach Anspruch 10, **dadurch gekennzeichnet,** daß die magnetische Flüssigkeit (14, 15) eine viskose organische Flüssigkeit ist.

12. Ventil nach einem der Ansprüche 1 bis 11, **dadurch gekennzeichnet,** daß die Dichtungsflüssigkeit (14, 15, 17, 20, 27) im Hinblick auf das Gas bzw. die Flüssigkeit, die durch das Ventil (13, 17, 24) strömen, inert ist.

13. Verwendung eines Ventils nach einem der Ansprüche 1 bis 12 bei einer implantablen Medikamentenpumpe (201), vorzugsweise einer Insulinpumpe für die Steuerung des Medikamentenflußes.

FIG 1

FIG 2

FIG 3

19

16

29

22

20

18

21

17

FIG 4

19

16

22

20

18

21

17

FIG 5

FIG 6

10

Europäisches
Patentamt

EUROPÄISCHER RECHERCHENBERICHT

Nummer der Anmeldung

EP 94 12 0725.0

# EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁶) |
|---|---|---|---|
| Y | Derwent's abstract, No K9799 E/33, week 8233, ABSTRACT OF SU, 872883 (BATURIN YUN), 25 Oktober 1981 (25.10.81) | 1-12 | F16J 15/53<br>F16K 1/34<br>F16K 13/10 |
| | -- | | |
| Y | EP, A2, 0117149 (FERROFLUIDICS CORPORATION), 29 August 1984 (29.08.84)<br>*Seite 3, Zeile 10 - Seite 5, Zeile 15, Figuren 1-6* | 1-12 | |
| | -- | | |
| A | US, A, 4883467 (FRANETZKI ET AL), 28 November 1989 (28.11.89)<br>*Figur 1, Zusammenfassung* | 1,13 | |
| | ----- | | RECHERCHIERTE SACHGEBIETE (Int. Cl.⁶) |
| | | | F16J<br>F16K |

Der vorliegende Recherchenbericht wurde für alle Patentansprüche erstellt.

| Recherchenort | Abschlußdatum der Recherche | Prüfer |
|---|---|---|
| STOCKHOLM | 24 April 1995 | WESTBERG CHRISTIAN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82